# EUROPÄISCHE PATENTSCHRIFT

(11) **EP 0 388 588 B1**
(45) Veröffentlichungstag und Bekanntmachung des Hinweises auf die Patenterteilung: **16.03.1994**
(21) Anmeldenummer: 90101144.5
(22) Anmeldetag: 20.01.1990
(51) Int. Cl.: C12N 11/04, C12N 11/10, C12G 1/06

(54) **Verfahren zur Herstellung immobilisierter Hefen**
Method for producing immobilized yeast
Procédé de préparation de levures immobilisées

(30) Priorität: 18.03.1989 DE 3908997
(43) Veröffentlichungstag der Anmeldung: 26.09.1990
(73) Patentinhaber: HÜLS AKTIENGESELLSCHAFT, 45764 Marl (DE)
(72) Erfinder: Hill, Frank, Dr., D-4020 Mettmann-Oberschwarzbach (DE)

(56) Entgegenhaltungen:
- EP-A- 0 173 915
- DE-A- 2 835 875
- DE-A- 3 613 575
- FR-A- 2 586 256
- CHEMICAL ABSTRACTS; Band 110, Nr. 13, 27. März 1989, S. 551, Zusammenfassung NR: 113113X; Columbus, Ohio, US; M.D. FUMI et al.
- FSTA (VITIS) DATABANK, Zusammenfassung Nr. 84-01-L0026 (QUEST AN = 84001428); PH. COULON et al.

## Beschreibung

Die Erfindung betrifft ein vereinfachtes Verfahren zur Herstellung von in Alginatperlen immobilisierten Hefen.

Zur Herstellung von alkoholischen Getränken, wie z. B. Bier, Wein oder Sekt, wird eine Gärung mit Hilfe von Hefen durchgeführt. Nach beendeter Gärung müssen die Hefen vollständig abgetrennt werden, wenn eine Trübung der Getränke vermieden werden soll.

Sekt wird durch eine zweite Gärung von aufgezuckertem Wein, dem besondere Hefestämme zugesetzt werden, hergestellt. Die Sektgärung erfolgt entweder in den handelsüblichen Sektflaschen, in größeren Spezialflaschen oder in Stahltanks. Bei der Flaschengärung ist die Abtrennung der Hefe nach Beendigung der Gärung aufwendig. Die schräg gelagerte Flasche wird über einen längeren Zeitraum wiederholt gerüttelt, bis der Hefetrub sich im Flaschenhals abgesetzt hat. Der Flaschenhals wird abgekühlt, wobei hier der Sekt mit der Hefe gefriert. Die Flasche wird dann geöffnet. Dabei wird der Eispfropfen mit dem Hefesediment durch den Innendruck herausgedrückt.

Dieses Verfahren kann nach FR 24 32 045 vereinfacht werden. Dort werden in einem Calciumalginatgel eingeschlossene Hefezellen verwendet, die sich ohne Rütteln im Flaschenhals absetzen.

Nach EP-A-0 173 915 werden Hefezellen zur Sektherstellung in Alginatperlen immobilisiert, worauf die Perlen noch mit einer zellenfreien Calciumalginatschicht umhüllt werden. Dadurch soll sichergestellt werden, daß keine Hefezellen aus dem Immobilisat freigesetzt werden.

In DE-C-28 35 875 wird ein aufwendiges Verfahren zur Herstellung von druckfesten Biokatalysatoren beschrieben. Dabei werden u. a. auch Alginatperlen mit Hefezellen verfestigt, mit physiologischer Kochsalzlösung gewaschen, getrocknet, geschrumpft und nachgehärtet. Ein Hinweis auf die Sektherstellung fehlt hier jedoch.

Werden Sekthefen in Calciumalginat immobilisiert, so werden entweder keine Vorkehrungen getroffen, oder es werden aufwendige Maßnahmen ergriffen, um einen Feintrub durch freigesetzte Hefezellen zu verhindern.

Aufgabe der vorliegenden Erfindung ist es, ein vereinfachtes Verfahren zur Herstellung von in Alginat immobilisierten Hefen bereitzustellen. Die immobilisierten Hefen sollen zur Herstellung alkoholischer Getränke geeignet sein. Dabei sollte im besonderen eine Flaschengärung von Sekt ohne Bildung von Feintrub sicher ermöglicht werden.

Die Aufgabe wird erfindungsgemäß dadurch gelöst, daß man die nach bekannten Verfahren hergestellten Calciumalginatperlen, in denen die Hefen immobilisiert sind, 30 bis 180 Minuten in einer CaCl₂-Lösung aushärtet, anschließend mit Wasser, das einen Salzgehalt von bis zu 0,5 g/l aufweisen kann, 100 bis 500 Minuten bei 5 bis 35 °C wäscht und dann bei 10 bis 50 °C Massetemperatur trocknet.

Zur Herstellung der hefehaltigen Calciumalginatperlen setzt man gewöhnlich eine 0,5- bis 5%ige Natriumalginatlösung ein, in der man die Hefe suspendiert. Diese Suspension wird dann in ein Fällungsbad, das im allgemeinen eine 0,5- bis 20%ige CaCl₂-Lösung ist, getropft. Dabei erhält man Perlen oder Granulat mit Durchmessern von 1 bis 6 mm.

Der Biomassegehalt der erfindungsgemäß hergestellten Perlen liegt meist im Bereich von 15 bis 80 % und der Alginatgehalt bei 80 bis 15 %. Die Restfeuchte beträgt 5 bis 20 %.

Geeignete Hefen für die Sektherstellung sind beispielsweise Saccharomyces bayanus oder Saccharomyces cerevisiae.

Die Perlen bleiben üblicherweise zur Aushärtung im Fällungsbad, bevor sie feucht entnommen werden und mit einer ausreichenden Menge Wasser gewaschen werden. Das Waschen kann in einem Kolben oder Kessel unter Schütteln oder Rühren erfolgen. Dabei wird allgemein ein 10- bis 300facher Wasserüberschuß, bezogen auf die feuchten Perlen, angewandt. Bei geringerem Überschuß ist die Waschung nicht immer erfolgreich. Man kann zwar auch bei einem mehr als 300fachen Überschuß waschen, derartige Überschüsse sind jedoch unnötig und unwirtschaftlich.

Man kann auch unter fließendem Wasser waschen.

Die Waschzeit beträgt vorzugsweise 120 bis 300 Minuten. Vorzugsweise wird bei 10 bis 30 °C gewaschen.

Zum Waschen wird praktisch keimfreies Nasser verwendet, wie beispielsweise Leitungswasser, Mineralwasser, entsalztes und vollentsalztes Wasser. Vorzugsweise wird mit vollentsalztem Wasser gewaschen.

Die Trocknung, die vorzugsweise bei 15 bis 40 °C Massetemperatur vorgenommen wird, kann beispielsweise in einem Umluftofen oder in einem Wirbelbett durchgeführt werden. Dabei kann auch vorgetrocknete Luft verwendet werden.

Vorzugsweise wird bis zu einem Trockensubstanzgehalt von 80 bis 95 % getrocknet.

Die so hergestellten Alginatperlen mit Hefen sind hervorragend zur Gärung von alkoholischen Getränken, wie beispielsweise Bier, Wein oder Sekt, geeignet. Nach der Gärung können die Perlen und dadurch auch die Hefen in einfacher Weise durch Dekantieren oder Filtration quantitativ abgetrennt werden. Die Getränke bleiben klar.

Die nach dem erfindungsgemäßen Verfahren hergestellten Calciumalginatperlen, in denen Sekthefe immobilisiert ist, können, kühl und trocken gelagert, auch nach einigen Monaten noch zur Sektherstellung gut eingesetzt werden. Für die Sektgärung werden dabei etwa 0,2 bis 2 g getrocknete Perlen auf 1 Liter aufgezuckerten Grundwein eingesetzt. Die Sektgärung ist bei 20 °C in 3 bis 8 Wochen abgeschlossen.

Als Grundwein eignet sich vor allem ein Wein mit einem Gehalt an freier Säure von bis zu 10 g/l, ausgedrückt als Weinsäure. Hier bleibt der Sekt nach der Sektgärung klar. Er zeigt weder eine Ausfällung an Kristallen noch einen Trub durch Hefezellen.

Die nach dem erfindungsgemäßen Verfahren hergestellten Calciumalginatperlen weisen einen Gehalt an auswaschbarem Calcium von 0,9 bis 1,6 mg/g auf.

Bei zu kurzer Wäsche liegt ein hoher Gehalt an auswaschbarem Calcium vor, so daß es bei der Sektgärung zu einer Trübung vor allem durch Kristallbildung (Calciumtartrat und -malat) kommt oder kommen kann. Bei einem zu geringen Gehalt an auswaschbarem Calcium ist das Netzwerk nicht mehr dicht genug, so daß schlecht sedimentierender Feintrub durch freigesetzte und sich vermehrende Zellen auftritt.

Nach dem erfindungsgemäßen Verfahren wird dagegen auf einfache Weise ein Immobilisat erhalten, mit dem eine Sektgärung durchgeführt werden kann, bei der weder eine Kristalltrübung noch ein Trub durch Hefezellen auftritt und bei der der Sekt deshalb sicher klar bleibt.

In den folgenden Beispielen werden die erfindungsgemäßen Beispiele durch Zahlen und die Vergleichsbeispiele durch Buchstaben gekennzeichnet.

### Beispiele

### Herstellung und Aushärtung der Calciumalginatperlen:

5 g Natriumalginat (PROTANAL^{R} LF 20/60, Fa. Protan, D-2000 Hamburg) werden in 100 ml Trinkwasser gestreut. Unter Rühren wird auf 90 °C erhitzt. Die Temperatur wird 1 Stunde bei 90 °C gehalten, wobei sich das Natriumalginat löst. Nachdem die Lösung auf 35 °C abgekühlt ist, werden 120 g Suspension frisch hergestellter Hefezellen des Stammes Saccharomyces bayanus (Trockensubstanzgehalt 5 %) zugegeben. In der resultierenden Mischung sind, bezogen auf den gesamten Trockensubstanzgehalt, 45 % Alginat und 55 % Biomasse enthalten.

Die klumpenfreie Suspension wird in ein Druckgefäß gegeben, dessen Boden mit 32 Düsennadeln von 0,5 mm innerem Durchmesser bestückt ist. Mit einem Druck von ca. 1,5 bar wird die Suspension durch die Düsennadeln gedrückt. Die von den Düsennadeln sich ablösenden Tropfen fallen nach ca. 10 cm in 600 ml einer schwach gerührten 2%igen CaCl₂-Lösung.

Nachdem die Suspension in 10 Minuten durchgedrückt ist, verbleiben die gebildeten Perlen zur Aushärtung noch 1 Stunde im Fällungsbad bei leichter Rührung.

### Wäsche und Trocknung:

Die gebildeten feuchten Perlen werden abgesiebt, 20 Sekunden mit Leitungswasser abgespült und dann in 7 Portionen von je 100 g in jeweils 10 Liter vollentsalztes Wasser gegeben, wo sie während einer Zeit gemäß Tabelle 1 unter leichtem Rühren gewaschen werden.

Die feuchten Perlen, die einen Durchmesser von etwa 2,5 mm und einen Trockensubstanzgehalt von 8 % aufweisen, werden abgesiebt und in einem Glasrohr mit einem Frittenboden durch Wirbelbetttrocknung getrocknet. Die dabei verwendete Luft wird auf 50 °C vorgewärmt. Die Trocknung wird beendet, wenn die Produkttemperatur auf 35 °C angestiegen ist. Die Perlen haben dann einen Trockensubstanzgehalt von 85 bis 88 % und sind auf einen Durchmesser von etwa 0,8 mm geschrumpft. die Gäraktivität hat sich im Vergleich zu den feuchten Zellen auf 30 bis 50 % vermindert.

Mit den Proben werden folgende Tests durchgeführt:
a) Gehalt an mit Wasser auswaschbarem Ca⁺⁺
1 g trockenes Granulat (Perlen) wird in 5 ml vollentsalztem Wasser 24 Stunden stehen gelassen. Anschließend wird abdekantiert und der im Wasser gelöste Ca⁺⁺-Gehalt bestimmt.
b) Bildung von Calciumtartrat/-malat im Grundwein und im aufgesäuerten Grundwein
100 ml eines trockenen Weißweines (7,9 % Ethanol, 0,2 % Glucose, 0,47 % Äpfelsäure, 0,18 % L-Weinsäure, pH 3,2) bzw. eines aufgesäuerten Grundweines, der aus obigem Weißwein durch Zugabe fester L-Weinsäure hergestellt wird und einem Weinsäuregehalt von 0,73 % aufweist, werden mit 1 g trockenem Hefeimmobilisat versetzt und dann in einem geschlossenen Gefäß 3 Wochen bei 20 °C stehen gelassen.
Das Immobilisat wird dann mit der Lupe auf Kristallwachstum an der Oberfläche untersucht.

**Tabelle 1**

| Bsp. | Dauer der Wäsche [min] | auswaschbares Ca⁺⁺ aus dem getrockneten Granulat [mg/g] | Kristallwachstum im | |
|---|---|---|---|---|
| | | | Grundwein | aufgesäuerten Grundwein |
| A | 10 | 2,8 | + | + |
| B | 30 | 2,0 | (+) | + |
| C | 60 | 1,8 | - | (+) |
| 1 | 120 | 1,5 | - | - |
| 2 | 240 | 1,3 | - | - |
| 3 | 360 | 1,1 | - | - |
| D | 600 | 0,7 | - | - |
| + : deutliche Kristallbildung (+): geringe Kristallbildung - : keine Kristallbildung | | | | |

c) Sektgärung
In einem trockenen Weißwein mit den oben aufgeführten Kennzahlen werden 24 g/l Saccharose gelöst. Je 0,7 Liter dieses aufgezuckerten Weines werden in 3 druckfeste Flaschen gefüllt, worauf 1,5 g Sekthefeimmobilisat gemäß Vergleichsbeispiel A, Beispiel 1 bzw. Vergleichsbeispiel D zugesetzt werden.
Die Flaschen werden mit Stopfen fest verschlossen und dann horizontal bei Raumtemperatur gelagert. Nach 4 Wochen werden die Flaschen auf 4 °C abgekühlt und geöffnet. Der Flascheninhalt wird untersucht.

**Tabelle 2**

| Immobilisat von Bsp. | Immobilisat nach der Gärung | Zuckergehalt im Sekt [g/l] | Geschmack des Sekts | sonstige Bemerkungen |
|---|---|---|---|---|
| A | durch Kristallbrücken fest verbacken | 2,0 | trocken, einwandfrei | Kristalltrübung |
| 1 | vereinzelt | 1,5 | trocken, einwandfrei | klar |
| D | vereinzelt | 4,8 | fast trocken, einwandfrei | leicht getrübt durch Hefezellen |

Nach Tabelle 1 wird bei den Beispielen 1 bis 3 und bei Vergleichsbeispiel D kein Kristallwachstum auf dem Immobilisat beobachtet. Aus Tabelle 2 geht hervor, daß mit dem Immobilisat von Beispiel 1, nicht aber mit dem von Vergleichsbeispiel D, ein einwandfreier Sekt hergestellt wird.

## Patentansprüche

1. Verfahren zur Herstellung von in Calciumalginatperlen immobilisierten Hefen, wobei man Hefezellen in einer Natriumalginatlösung in eine CaCl₂-Lösung tropft, die dabei entstandenen Perlen in der CaCl₂-Lösung aushärtet, mit Wasser wäscht und dann trocknet,
dadurch gekennzeichnet,
daß man
- 30 bis 180 Minuten aushärtet,
- mit Wasser mit einem Salzgehalt von bis zu 0,5 g/l 100 bis 500 Minuten bei 5 bis 35 °C wäscht
- und dann bei 10 bis 50 °C Massetemperatur trocknet.

2. Verfahren nach Anspruch 1,
dadurch gekennzeichnet,
daß man 120 bis 300 Minuten bei 10 bis 30 °C wäscht.

3. Verfahren nach Anspruch 2,
dadurch gekennzeichnet,
daß man mit vollentsalztem Wasser wäscht.

4. Verfahren nach Anspruch 1,
dadurch gekennzeichnet,
daß man bei 15 bis 40 °C Massetemperatur trocknet.

5. Verfahren nach Anspruch 1,
dadurch gekennzeichnet,
daß man bis zu einem Trockensubstanzgehalt von 80 bis 95 % trocknet.

6. Verwendung von immobilisierten Hefen gemäß Anspruch 1 zur Herstellung von alkoholischen Getränken.

7. Verwendung von immobilisierten Hefen gemäß Anspruch 6 zur Sektherstellung aus einem Grundwein mit einem Gehalt an freier Säure von bis zu 10 g/l, berechnet als Weinsäure.

## Claims

1. Process for the preparation of yeasts which are immobilized in calcium alginate beads, in which process yeast cells in a sodium alginate solution are added dropwise to a CaCl₂ solution and the resulting beads are hardened in the CaCl₂ solution, washed with water and then dried,
characterized in that
the beads
- are hardened for 30 to 180 minutes,
- washed with water having a salt content of up to 0.5 g/l for 100 to 500 minutes at 5 to 35°C
- and then dried at a product temperature of 10 to 50°C.

2. Process according to Claim 1,
characterized in that
the beads are washed for 120 to 300 minutes at 10 to 30°C.

3. Process according to Claim 2,
characterized in that
the beads are washed with fully demineralized water.

4. Process according to Claim 1,
characterized in that
the beads are dried at a product temperature of 15 to 40°C.

5. Process according to Claim 1,
characterized in that
the beads are dried until their dry matter content is 80 to 95%.

6. Use of immobilized yeasts according to Claim 1 for the production of alcoholic beverages.

7. Use of immobilized yeasts according to Claim 6 for the production of sparkling wine from a base wine with a free acid content of up to 10 g/l, calculated as tartaric acid.

## Revendications

1. Procédé pour la préparation de levures immobilisées dans des perles d'alginate de calcium, dans lequel on verse goutte à goutte, dans une solution de chlorure de calcium, des cellules de levure dans une solution d'alginate de sodium, durcit les perles qui se sont alors formées dans la solution de chlorure de calcium, les lave à l'eau et les sèche ensuite,
caractérisé par le fait :
- que l'on durcit pendant 30 à 180 minutes,
- que l'on lave pendant 100 à 500 minutes, à une température de 5 à 35°C, avec de l'eau d'une teneur en sel allant jusqu'à 0,5 g par litre, et
- que l'on sèche ensuite à une température de masse de 10 à 50°C.

2. Procédé selon la revendication 1,
caractérisé par le fait que l'on lave pendant 120 à 300 minutes à une température de 10 à 30°C,

3. Procédé selon la revendication 2,
caractérisé par le fait que l'on lave avec de l'eau totalement déminéralisée.

4. Procédé selon la revendication 1,
caractérisé par le fait que l'on sèche à une température de masse de 15 à 40°C.

5. Procédé selon la revendication 1,
caractérisé par le fait que l'on sèche jusqu'à une teneur en substance sèche de 80 à 95 %.

6. L'utilisation de levures immobilisées selon la revendication 1, pour la préparation de boissons alcoolisées.

7. L'utilisation de levures immobilisées selon la revendication 6, pour préparer un vin mousseux à partir d'un vin de base d'une teneur en acide libre allant jusqu'à 10 grammes par litre, calculée à l'état d'acide tartrique.
